(19) Europäisches Patentamt
European Patent Office
Office européen des brevets

(11) Veröffentlichungsnummer: **0 260 675**
**A1**

# (12) EUROPÄISCHE PATENTANMELDUNG

(21) Anmeldenummer: **87113539.8**

(22) Anmeldetag: **16.09.87**

(51) Int. Cl.⁴: **C07D 211/90**, C07D 401/12, C07D 405/12, A61K 31/44, A61K 31/455

Claims for the following Contracting States: AT + ES.

(30) Priorität: **16.09.86 HU 394886**

(43) Veröffentlichungstag der Anmeldung: **23.03.88 Patentblatt 88/12**

(84) Benannte Vertragsstaaten: **AT BE CH DE ES FR GB IT LI NL SE**

(71) Anmelder: **ALKALOIDA VEGYéSZETI GYáR**
**Kabay János ut 27-29**
**H-4440 Tiszavasvári(HU)**

(72) Erfinder: **Szilágyi, Géza, Dr.**
**Szakasits A. u. 66/a**
**H-1115 Budapest(HU)**
Erfinder: **Bozó, Eva**
**VIII, u. 22**
**H-1172 Budapest(HU)**
Erfinder: **Czollner, László**
**Arpád u. 169**
**H-1042 Budapest(HU)**
Erfinder: **Jaszlits, Lászlo**
**Maros u. 4**
**H-1122 Budapest(HU)**
Erfinder: **Rabloczky, György, Dr.**
**Battyány u. 3**
**H-1015 Budapest(HU)**
Erfinder: **Borsi, József, Dr.**
**Bartók B. u. 90**
**H-1113 Budapest(HU)**
Erfinder: **Elekes, István, Dr.**
**Delej u. 28**
**H-1089 Budapest(HU)**
Erfinder: **Nagy geb. Csókás, Gyöngyi**
**Barók B. u. 88**
**H-1113 Budapest(HU)**
Erfinder: **Varró, András, Dr.**
**Fadrusz u. 2**
**H-1114 Budapest(HU)**
Erfinder: **Láng, Zsuzsa, Dr.**
**Keselyü u. 1**
**H-1025 Budapest(HU)**
Erfinder: **Cseh, György, Dr.**
**Népszinház u. 33**
**H-1081 Budapest(HU)**
Erfinder: **Horváth, Gyula, Dr.**
**Kigyó u. 2-4**
**H-1052 Budapest(HU)**

EP 0 260 675 A1

Erfinder: **Bódi, Ilona, Dr.**
**Szálloda u. 2/a**
**H-3300 Eger(HU)**

(74) Vertreter: **Beszédes, Stephan G. Dr.**
**Münchener Strasse 80a Postfach 1168**
**D-8060 Dachau(DE)**

(54) **4-[N-(Substituierte)-amino-phenyl]-substituierte 1,4-Di-(hydro)-pyridinderivate, Verfahren zu ihrer Herstellung und diese enthaltende Arzneimittel.**

(57) Gegenstand der Erfindung sind 4-[N-(substituierte)-aminophenyl]-substituierte 1,4-Di-(hydro)-pyridinderivate der allgemeinen Formel

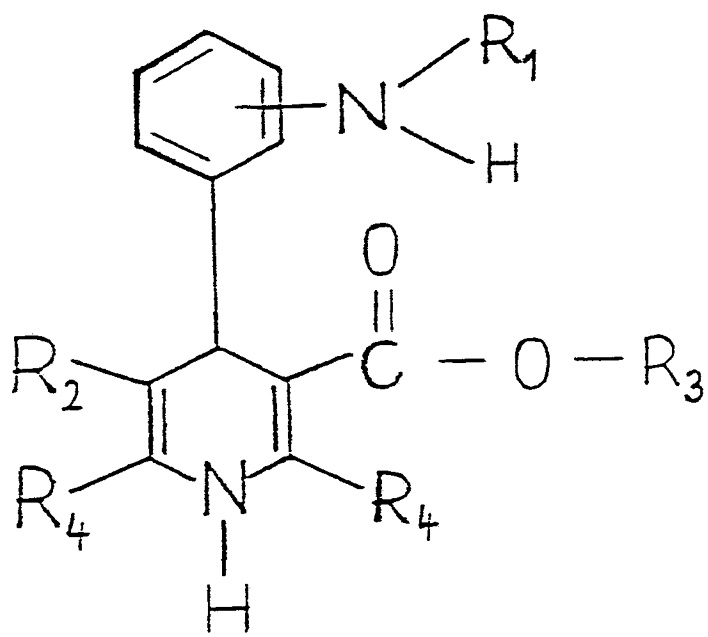

I ,

worin
$R_1$
für einen Alkanoylrest, einen Cycloalkanoylrest, einen Arylcarbonyl-beziehungsweise Aralkylencarbonylrest der allgemeinen Formel II, einen Trifluoracetylrest, einen Alkoxycarbonylrest der allgemeinen Formel III, einen Alkylsulfonyl-oder Phenylsulfonylrest der allgemeinen Formel IV steht,
$R_2$
eine Cyangruppe oder einen Alkoxycarbonylrest bedeutet,
$R_3$
für einen Alkylrest, einen Cycloalkylrest oder einen Alkoxyalkylrest steht und
$R_4$
einen Alkylrest bedeutet,
einschließlich ihrer racemischen und optisch aktiven Formen und Gemische sowie ihre Säureadditionssalze.

Gegenstand der Erfindung sind auch ein Verfahren zur Herstellung dieser Verbindungen und diese enthaltende Arzneimittel.

**4-[N-(Substituierte)-aminophenyl]-substituierte 1,4-Di-(hydro)-pyridinderivate, Verfahren zu ihrer Herstellung und diese enthaltende Arzneimittel**

Die Erfindung betrifft neue 4-[N-(substituierte)-aminophenyl]-substituierte 1,4-Di-(hydro)-pyridinderivate, ein Verfahren zu ihrer Herstellung und diese Verbindungen enthaltende Arzneimittel, insbesondere mit Calciumantagonistwirkung, antihypertensiver Wirkung und ferner die Gebärmutter entspannender Wirkung.

Es ist bekannt, daß substituierte 1,4-Di-(hydro)-pyridinderivate günstige pharmakologische Eigenschaften, welche beispielsweise in den europäischen Patentschriften 88 903, 94 159 und 106 276 erörtert sind, haben. Die neuen erfindungsgemäßen Verbindungen der allgemeinen Formel I, welche im Gegensatz zu den bekannten Verbindungen in der vierten Stelle des Dihydropyridin-Restes einen durch Amidgruppe substituierten Phenylring besitzen, weisen höchst wertvolle Eigenschaften auf, welche diejenigen der bekannten Verbindungen übertreffen.

Der Erfindung liegt die Aufgabe zugrunde, überlegene therapeutische Wirkungen, insbesondere eine überlegene Calciumantagonistwirkung und eine antihypertensive Wirkung und auch eine die Gebärmutter entspannende Wirkung aufweisende neue 1,4-Di-(hydro)-pyridinderivate, ein Verfahren zu ihrer Herstellung und diese Verbindungen enthaltende Arzneimittel zu schaffen.

Das Obige wurde überraschenderweise durch die Erfindung erreicht.

Gegenstand der Erfindung sind 4-[N-(substituierte)-aminophenyl]-substituierte 1,4-Di-(hydro)-pyridinderivate der allgemeinen Formel

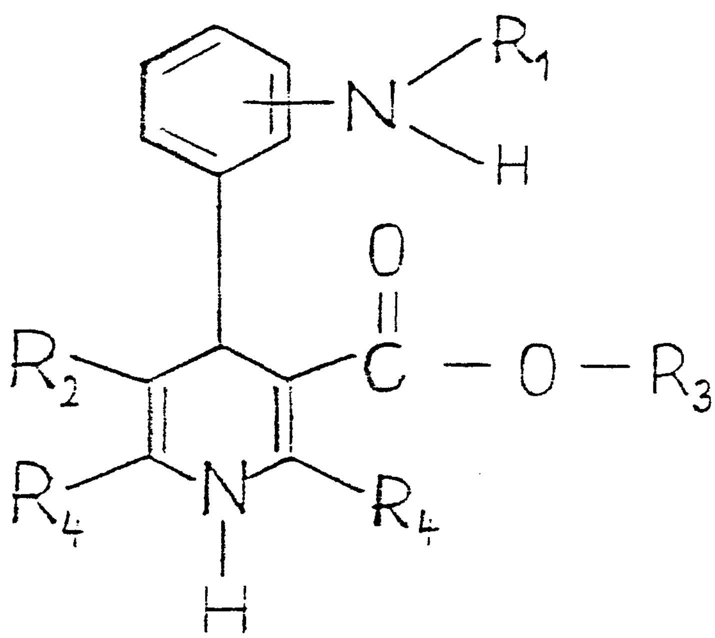

I ,

worin

$R_1$

für einen Alkanoylrest mit 2 bis 10 Kohlenstoffatomen, einen Cycloalkanoylrest mit 4 bis 7 Kohlenstoffatomen, einen Arylcarbonyl-beziehungsweise Aralkylencarbonylrest der allgemeinen Formel

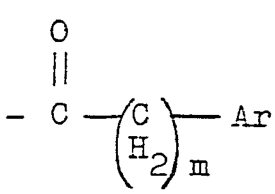

II ,

in welchletzterer

m

0 bis 3 ist,

die Alkylengruppe

$$-(CH_2)_m-$$

geradkettig oder verzweigt, gesättigt oder ungesättigt ist und

Ar

einen, gegebenenfalls durch ein Halogenatom, einen Alkylrest mit 1 bis 4 Kohlenstoffatom(en), 1 oder mehrere Alkoxyrest(e) mit 1 bis 4 Kohlenstoffatom(en), einen Alkylthiorest mit 1 bis 4 Kohlenstoffatom(en), einen Alkylsulfinylrest mit 1 bis 4 Kohlenstoffatom(en), einen Alkylsulfonylrest mit 1 bis 4 Kohlenstoffatom-(en), einen Trifluormethylrest, eine Nitrogruppe, eine Aminogruppe oder eine, gegebenenfalls durch einen Acetyl-, Trifluoracetyl-oder einen Methansulfonylrest substituierte, Aminogruppe substituierten, Arylrest oder einen ein Stickstoff-und/oder Sauerstoffatom aufweisenden aromatischen Rest darstellt,

einen Trifluoracetylrest, einen Alkoxycarbonylrest der allgemeinen Formel

$$-\overset{\displaystyle O}{\overset{\|}{C}}-O-R_5 \quad III,$$

in welchletzterer

$R_5$

einen Alkylrest mit 1 bis 4 Kohlenstoffatom(en) darstellt, oder

einen Alkylsulfonyl-oder Phenylsulfonylrest der allgemeinen Formel

$$-\underset{O_2}{S}-R_6 \quad IV,$$

in welchletzterer

$R_6$

einen Alkylrest mit 1 bis 4 Kohlenstoffatom(en), oder einen, gegebenenfalls durch einen Alkylrest mit 1 bis 4 Kohlenstoffatom(en) oder eine Nitrogruppe substituierten, Phenylrest darstellt,

steht,

$R_2$

eine Cyangruppe oder einen geradkettigen oder verzweigten Alkoxycarbonylrest mit 2 bis 4 Kohlenstoffatomen bedeutet,

$R_3$

für einen geradkettigen oder verzweigten Alkylrest mit 1 bis 4 Kohlenstoffatom(en), einen Cycloalkylrest mit 5 bis 7 Kohlenstoffatomen oder einen Alkoxyalkylrest mit 2 bis 6 Kohlenstoffatomen steht und

$R_4$

einen Alkylrest mit 1 bis 4 Kohlenstoffatomen bedeutet,

einschließlich ihrer racemischen und optisch aktiven Formen und Gemische sowie ihre Säureadditionssalze.

Es ist bevorzugt, daß der Alkanoylrest, für den $R_1$ stehen kann, ein solcher mit 2 bis 9, insbesondere 2, 3, 4 oder 9, Kohlenstoffatomen ist, beziehungsweise der Cycloalkanoylrest, für den $R_1$ stehen kann, ein solcher mit 4 bis 6, insbesondere 4 oder 5, ganz besonders 4, Kohlenstoffatomen ist, beziehungsweise der Arylcarbonylbeziehungsweise Aralkylencarbonylrest der allgemeinen Formel II, für den $R_1$ stehen kann, ein solcher, bei welchem m 0 bis 2, insbesondere 0 oder 1, ist und/oder der Arylrest, für den Ar stehen kann, ein Phenylrest ist und/oder ein solcher, bei welchem der Alkylrest, der beziehungsweise die Alkoxyrest(e), der Alkylthiorest, der Alkylsulfinylrest beziehungsweise der Alkylsulfonylrest, durch welche[n] er substituiert sein kann, ein solche[r] mit 1 oder 2, insbesondere 1, Kohlenstoffatom(en) ist beziehungsweise sind, beziehungsweise der ein Stickstoff-und/oder Sauerstoffatom aufweisende aromatische Rest, für den Ar stehen kann, ein solcher mit 5 oder 6 Ringgliedern, insbesondere ein Furyl-oder Pyridyl-, ganz besonders Pyrid-3-yl-oder Pyrid-4-ylrest, ist, beziehungsweise der Alkoxycarbonylrest der allgemeinen Formel III, für den $R_1$ stehen kann, ein solcher, dessen $R_5$ einen Alkylrest mit 1 oder 2, insbesondere 1, Kohlenstoffatom-(en) darstellt, ist, beziehungsweise der Alkylsulfonylrest der allgemeinen Formel IV, für den $R_1$ stehen kann, ein solcher, bei welchem der Alkylrest, für den $R_6$ stehen kann, ein solcher mit 1 oder 2, insbesondere 1, Kohlenstoffatom(en) ist, beziehungsweise der Phenylsulfonylrest der allgemeinen Formel IV, für den $R_1$ stehen kann, ein solcher, bei welchem der Alkylrest, durch den der Phenylrest, für den $R_6$ stehen kann, substituiert sein kann, ein solcher mit 1 oder 2, insbesondere 1, Kohlenstoffatom(en) ist.

Ferner ist es bevorzugt, daß der Alkoxycarbonylrest, für den $R_2$ stehen kann, ein solcher mit 2 oder 3, insbesondere 2, Kohlenstoffatomen ist.

Es ist auch bevorzugt, daß der beziehungsweise die Alkylrest(e), für welche[n] $R_3$ und/oder $R_4$ stehen kann beziehungsweise können, ein solche[r] mit 1 bis 3, insbesondere 1 oder 2, ganz besonders 1, Kohlenstoffatom(en) ist beziehungsweise sind, beziehungsweise der Cycloalkylrest, für den $R_3$ stehen kann, ein solcher mit 5 oder 6, insbesondere 6, Kohlenstoffatomen ist, beziehungsweise der Alkoxyalkylrest, für den $R_3$ stehen kann, ein solcher mit 2 oder 3, insbesondere 3, Kohlenstoffatomen, ganz besonders ein Methoxyäthylrest, vor allem ein 2-(Methoxy)-äthylrest, ist.

Sehr bevorzugte Reste, für die $R_1$ stehen kann, sind Nitrobenzoyl-, vor allem 4-(Nitro)-benzoyl-, Methylbenzoyl-, vor allem 4-(Methyl)-benzoyl-, Äthylbenzoyl-, vor allem 4-(Äthyl)-benzoyl-, und Trifluoracetylreste.

Sofern es sich beim Rest der allgemeinen Formel II um einen mit einem ungesättigten Alkylenrest handelt, ist m bevorzugt 2.

Vorzugsweise ist die substituierte Aminogruppe in der 2-oder 3-Stellung, insbesondere in der 2-Stellung, des in der 4-Stellung des 1,4-Di-(hydro)-pyridinringes befindlichen Phenylrestes.

Sofern im Arylcarbonyl-beziehungsweise Aralkylencarbonylrest der allgemeinen Formel II, für welchen $R_1$ stehen kann, der Arylrest, für den Ar stehen kann, durch mehrere Alkoxyreste substituiert ist, ist deren Zahl vorzugsweise 2 oder 3. Falls der Arylrest, für den Ar stehen kann, ein Phenylrest ist, sind diese Alkoxyreste vorzugsweise in den 3-und 4-beziehungsweise 3-, 4-und 5-Stellungen des Phenylrestes.

Eine bevorzugte Gruppe der erfindungsgemäßen 1,4-Di-(hydro)-pyridinderivate sind diejenigen, bei welchen

$R_1$

für einen Alkanoylrest mit 2 bis 10 Kohlenstoffatomen, insbesondere einen Acetyl-oder Octanoylrest, oder einen Cycloalkanoylrest mit 4 bis 7 Kohlenstoffatomen, insbesondere einen Cyclopropancarbonylrest, steht und

$R_2$ , $R_3$ und $R_4$

die oben angegebenen Bedeutungen haben.

Dabei sind als $R_2$ der Methoxycarbonyl-oder Äthoxycarbonylrest besonders bevorzugt, als $R_3$ ein Alkylrest mit 1 bis 4 Kohlenstoffatom(en), insbesondere der Methyl-oder Äthylrest, bevorzugt und als $R_4$ ein Methylrest besonders bevorzugt.

Eine weitere bevorzugte Gruppe der erfindungsgemäßen 1,4-Di-(hydro)-pyridinderivate sind diejenigen, bei welchen

$R_1$

für einen Rest der allgemeinen Formel II,

in welchletzterer

Ar

einen, gegebenenfalls durch ein Halogenatom, einen Alkylrest mit 1 bis 4 Kohlenstoffatom(en), 1 oder mehrere Alkoxyrest(e) mit 1 bis 4 Kohlenstoffatom(en), einen Alkylthiorest mit 1 bis 4 Kohlenstoffatom(en), einen Alkylsulfinylrest mit 1 bis 4 Kohlenstoffatom(en), einen Alkylsulfonylrest mit 1 bis 4 Kohlenstoffatom-(en), einen Trifluormethylrest, eine Nitrogruppe, eine Aminogruppe oder eine, gegebenenfalls durch einen Acetyl-, Trifluoracetyl-oder einen Methansulfonylrest substituierte, Aminogruppe substituierten, Phenylrest oder einen ein Stickstoff-und/oder Sauerstoffatom aufweisenden aromatischen Rest darstellt und

m, $R_2$ , $R_3$ und $R_4$

die oben angegebenen Bedeutungen haben.

Dabei sind als $R_1$ ein Arylcarbonyl-beziehungsweise Aralkylencarbonylrest der allgemeinen Formel II, bei welchem der Alkylrest, durch den der Arylrest, für den Ar stehen kann, substituiert sein kann, der Methyl-oder Äthylrest ist, der beziehungsweise die Alkoxyrest(e), durch den beziehungsweise die der Arylrest, für den Ar stehen kann, substituiert sein kann, [ein] Methoxyrest(e) ist beziehungsweise sind, der Alkylthiorest, durch den der Arylrest, für den Ar stehen kann, substituiert sein kann, der Methylthiorest ist, der Alkylsulfinylrest, durch den der Arylrest, für den Ar stehen kann, substituiert sein kann, der Methylsulfinylrest ist beziehungsweise der Alkylsulfonylrest, durch den der Arylrest, für den Ar stehen kann, substituiert sein kann, der Methylsulfonylrest ist beziehungsweise der ein Stickstoff-und/oder Sauerstoffatom aufweisende aromatische Rest ein Furoyl-, Nicotinoyl-oder Isonicotinoylrest ist, besonders bevorzugt, als $R_2$, sofern es für einen Alkoxycarbonylrest steht, der Methoxycarbonyl-, Äthoxycarbonyl-, n-Propoxycarbonyl-, Isopropoxycarbonyl-oder tert.-Butoxycarbonylrest besonders bevorzugt, als $R_3$ der Methyl-, Äthyl-, Cyclohexyl-oder 2-(Methoxy)-äthylrest besonders bevorzugt und als $R_4$ der Methylrest besonders bevorzugt.

Eine andere bevorzugte Gruppe der erfindungsgemäßen 1,4-Di-(hydro)-pyridinderivate sind diejenigen, bei welchen

$R_1$

für einen Trifluoracetylrest steht und

$R_2$, $R_3$ und $R_4$
die oben angegebenen Bedeutungen haben.
Dabei sind als $R_2$, sofern es für einen Alkoxycarbonylrest steht, der Methoxycarbonylrest besonders bevorzugt, als $R_3$ der Methyl-, Äthyl-, Cyclohexyl-oder 2-(Methoxy)-äthylrest besonders bevorzugt und als $R_4$ der Methylrest besonders bevorzugt.

Ganz besonders bevorzugte erfindungsgemäße 1,4-Di-(hydro)-pyridinderivate sind
2,6-Di-[methyl]-3,5-bis-[methoxycarbonyl]-4-[2'-(4"-<nitro> -benzamido)-phenyl]-1,4-di-[hydro]-pyridin,
2,6-Di-[methyl]-3,5-bis-[methoxycarbonyl]-4-(2'-(4"-<methyl> -benzamido)-phenyl]-1,4-di-[hydro]-pyridin,
2,6-Di-[methyl]-3,5-bis-[methoxycarbonyl]-4-[2'-(4"-<äthyl> -benzamido)-phenyl]-1,4-di-[hydro]-pyridin und
2,6-Di-[methyl]-3,5-bis-[methoxycarbonyl]-4-[2'-(trifluoracetamido)-phenyl]-1,4-di-[hydro]-pyridin
einschließlich ihrer racemischen und optisch aktiven Formen und Gemische sowie ihre Säureadditionssalze.

Gegenstand der Erfindung ist auch ein Verfahren zur Herstellung der erfindungsgemäßen 1,4-Di-(hydro)-pyridinderivate, welches dadurch gekennzeichnet ist, daß 4-(aminophenyl)-substituierte 1,4-Di-(hydro)-pyridinderivate der allgemeinen Formel

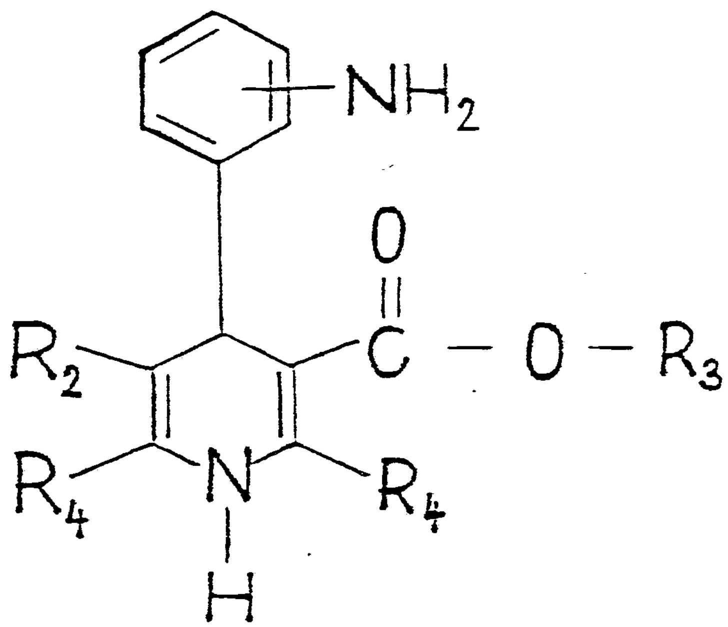

V ,

worin $R_2$, $R_3$ und $R_4$ die oben angegebenen Bedeutungen haben, mit Verbindungen der allgemeinen Formel
$R_1$ -X VI ,
worin
$R_1$
die oben angegebenen Bedeutungen hat und
X
eine zum Einführen einer Gruppe $R_1$ in das Molekül geeignete abspaltbare Gruppe darstellt,
umgesetzt werden und gegebenenfalls die erhaltenen 1,4-Di-(hydro)-pyridinderivate der allgemeinen Formel I, bei welchen $R_1$ für einen Arylcarbonylrest der allgemeinen Formel II mit m = 0, bei welchem Ar einen durch eine Nitrogruppe substituierten Arylrest bedeutet, steht, in an sich bekannter Weise reduziert werden, worauf in an sich bekannter Weise gegebenenfalls die erhaltenen 1,4-Di-(hydro)-pyridinderivate der allgemeinen Formel I in ihre Säureadditionssalze überführt werden und/oder gegebenenfalls die erhaltenen Säureadditionssalze der 1,4-Di-(hydro)-pyridinderivate der allgemeinen Formel I in die entsprechenden freien Basen und/oder andere Säureadditionssalze überführt werden und/oder gegebenenfalls die erhaltenen 1,4-Di-(hydro)-pyridinderivate der allgemeinen Formel I beziehungsweise ihre Säureadditionssalze in ihre optisch aktiven Formen gespalten werden und/oder gegebenenfalls die optisch aktiven Antipoden der 1,4-Di-(hydro)-pyridinderivate der allgemeinen Formel I beziehungsweise ihrer Säureadditionssalze racemisiert werden.

Gemäß der im Schrifttum angenommenen Definition (T. A. Geissman: Principles of Organic Chemistry, Ed. 3, Editor W. H. Freeman, London, 1968) ist unter "abspaltbarer Gruppe" X eine solche, welche gegen ein nukleophiles Reagens leicht austauschbar ist, zu verstehen. Solche sind Halogenatome, insbesondere Chlor-, Brom-und Jodatome, und außerdem Acetoxy-und Trifluoracetoxygruppen. Im erfindungsgemäßen Verfahren ist Chlor eine besonders bevorzugte "abspaltbare Gruppe" X.

Gemäß einer vorteilhaften Ausführungsform des erfindungsgemäßen Verfahrens werden 1,1 bis 2,5 Mol einer Verbindung der allgemeinen Formel VI mit einer m Lösung eines 4-(aminophenyl)-substituierten 1,4-Di-(hydro)-pyridinderivates der allgemeinen Formel V in einem organischen Lösungsmittel, zweckmäßig Dioxan oder Benzol, bei Temperaturen von 0 bis 30°C, zweckmäßig 5 bis 15°C, gegebenenfalls in Gegenwart von 1,1 bis 2 Mol einer organischen Base, wie Pyridin, versetzt und das Reaktionsgemisch wird 1 bis 20 Stunden lang bei Raumtemperatur gerührt beziehungsweise geschüttelt. Nach beendeter Reaktion wird das Reaktionsgemisch in Wasser eingegossen, der pH-Wert mit einer Säure neutralisiert und der ausgeschiedene Niederschlag abfiltriert und gegebenenfalls durch Umkristallisieren gereinigt. Alkohole mit 1 bis 4 Kohlenstoffatom(en), wie Äthanol, sind vorteilhafte Umkristallisierungslösungsmittel.

Als vorteilhafte Verbindungen der allgemeinen Formel VI werden Säurechloride, Säureanhydride, Dialkylpyrocarbonate oder Chlorameisensäureester eingesetzt.

Die Reduktion der 1,4-Di-(hydro)-pyridinderivate der allgemeinen Formel I, bei welchen $R_1$ für einen Arylcarbonylrest der allgemeinen Formel II mit m = 0, bei welchem Ar einen durch eine Nitrogruppe substituierten Arylrest, zum Beispiel Nitrophenylrest, bedeutet, kann vorteilhaft durch katalytische Hydrogenolyse in Alkoholen mit 1 bis 4 Kohlenstoffatom(en) bei Raumtemperatur und unter Atmosphärendruck mit Palladium auf Kohle [Pd-C] als Katalysator durchgeführt werden. Diese Reduktion kann auch, im Falle daß m von 0 verschieden ist, entsprechend durchgeführt werden.

Die erfindungsgemäßen 1,4-Di-(hydro)-pyridinderivate der allgemeinen Formel I, welche einen basischen Stickstoffsubstituenten aufweisen, können in protischen Lösungsmitteln, wie Isopropanol, mit organischen oder anorganischen Säuren, wie Salzsäure, Maleinsäure oder Fumarsäure, in Säureadditionssalze überführt werden. Diese Salzherstellung kann nach an sich bekannten Verfahrensweise durchgeführt werden. So kann die 1,4-Di-(hydro)-pyridinbase in einem Alkohol gelöst und dann mit der ge wünschten Säure oder einer Lösung derselben versetzt werden. Das erhaltene Salz kann entweder abfiltriert oder durch Abdampfen des Lösungsmittels isoliert werden. Gegebenenfalls kann es durch Umkristallisieren gereinigt werden.

Die im erfindungsgemäßen Verfahren als Ausgangssubstanzen eingesetzten 4-(aminophenyl)-substituierten 1,4-Di-(hydro)-pyridinderivate der allgemeinen Formel V sind zum Teil bekannte Stoffe (J. Am. Chem. Soc., 71, [1949], 4 003; Südafrikanische Patentschrift 68 01482) oder können in analoger Weise hergestellt worden sein.

Die Verbindungen der allgemeinen Formel VI sind handelsüblich.

Gegenstand der Erfindung sind auch Arzneimittel, welche durch einen Gehalt an 1 oder mehr erfindungsgemäßen 1,4-Di-(hydro)-pyridinderivaten als Wirkstoff(en), zweckmäßig zusammen mit 1 oder mehr pharmazeutisch üblichen Träger-und/oder Hilfsstoff(en), gekennzeichnet sind.

Die erfindungsgemäßen 1,4-Di-(hydro)-pyridinderivate haben nämlich wie bereits gesagt wertvolle therapeutische Wirkungen, insbesondere eine Calciumantagonistwirkung und antihypertensive Wirkung. Diese günstige Wirkung äußert sich in direkter koronarer, cerebraler und peripherer gefäßerweiternder Wirksamkeit. Daher ist ihre Anwendung zur Therapie von cerebralen, cardialen und peripheren Gefäßerkrankungen sowie cardialer Ischämie, Gehirnblutungen, Gehirninfarkt, vorübergehender Dysfunktion der Gehirngefäße, Atherosklerose, Nierenatherosklerose und anderer Stenosen angezeigt.

Im Gegensatz zu Beta-Rezeptoren-Blockern haben diese Verbindungen die günstige Eigenschaft, daß sie auch bronchokonstriktive Wirksamkeit aufweisen, sie können also auch bei Asthmapatienten eingesetzt werden.

Ihre Schutzwirkung auf die Gefäße, besonders auf die des Herzens, ist von größter Bedeutung. Sie ermöglicht es, sie zur Heilung von Gefäßerkrankungen und Stenokardie anzuwenden.

Sie übertreffen außerdem Arzneimittel ähnlicher Indikation, da sie keine negativ inotrope Nebenwirkungen ausüben.

Die erfindungsgemäßen 1,4-Di-(hydro)-pyridinderivate haben auch eine die Gebärmutter entspannende Wirkung. In der isolierten Rattengebärmutter hemmen sie in niedriger Konzentration die mit $PGF_{2alpha}$ hervorgerufene Gebärmutterkontraktion.

Gebärmutter entspannende Wirkung in der isolierten Rattengebärmutter in vitro

Der Test wurde gemäss der Methode von Gaddum und Hammeed [Brit. J. Pharmacol., 9, 240 (1954)] durchge führt. Die Hemmwirkung von $10^{-6}$ M Dosen der Testverbindungen auf die mit $PGF_{2alpha}$ hervorgerufene Gebärmutterkontraktion wurde gemessen. Nifedipin {2,6-Di-[methyl]-4-[2'-(nitro)-phenyl]-1,4-di-[hydro]-pyridin-3,5-di-[carbonsäuredimethylester]} diente als Vergleichssubstanz. Die Ergebnisse, die in Tabelle 1 zusammengefaßt sind, zeigen, daß die erfindungsgemäßen Verbindungen gleiche Aktivität wie die Vergleichssubstanz haben.

Tabelle 1

Hemmwirkung auf $PGF_{2alpha}$ der Verbindungen der allgemeinen Formel (I)

| Verbindung Beispiel Nr. | Konzentration der Prüfsubstanz Mol | Hemmung der durch $PGF_{2alpha}$ hervorgerufenen Kontraktion % |
|---|---|---|
| 8 | $10^{-6}$ | 96,7 |
| 9 | $10^{-6}$ | 94,3 |
| 10 | $10^{-6}$ | 94,0 |
| 11 | $10^{-6}$ | 77,8 |
| 12 | $10^{-6}$ | 83,3 |
| 34 | $10^{-6}$ | 98,0 |
| 42 | $10^{-6}$ | 88,0 |
| Nifedipin | $10^{-6}$ | 95,0 |

Die antihypertensive Wirkung der erfindungsgemässen Verbindungen der allgemeinen Formel I wurde mit den folgenden Prüfmethoden in vivo bestimmt.

Antihypertensive Wirkung an narkotisierten Katzen

Um spontane Atmung zu sichern, wurde unter Pentobarbital-Natrium-Narkose (35 mg/kg i.p.) ein Katheter in die Trachea der Tiere eingeführt. An einer Seite wurden die Oberschenkelarterie und -vene katheterisiert. Die vegetativen Prüfsubstanzen (Adrenalin, Isoproterenol) wurden durch den Venenkatheter verabreicht. Der arterielle Durchschnittsblutdruck wurde durch den Oberschenkelarterienkatheter, der mit einem Statham P23Db Drucküberträger und Elektromanometer verbunden war, gemessen. Der Puls wurde mit einem durch arterielle Pulswellen gesteuerten Kardiotachometer bestimmt.

Die verschiedenen Prüfsubstanzen wurden durch einen in das Duodenum eingeführten Katheter verabreicht. Die Ergebnisse sind in Tabelle 2 zusammengefasst.

Tabelle 2

Hypotensive Wirkung der Verbindungen der allgemeinen Formel I an narkotisierten, normotensiven Katzen. Dosis: 1 mg/kg id.

| Beispiel Nr. | Arterieller Durchschnittsblutdruck (mm Hg) | | | | | |
|---|---|---|---|---|---|---|
| | 0 Min. | 5 Min. | 15 Min. | 30 Min. | 60 Min. | 120 Min. |
| 6 | 120 | 115 | 85 | 65 | 70 | 75 |
| 7 | 145 | 115 | 60 | 50 | 60 | 85 |
| 9 | 135 | 125 | 85 | 65 | 75 | 102 |
| 10 | 140 | 60 | 70 | 80 | 85 | - |
| 11 | 152 | 147 | 130 | 125 | 120 | 132 |
| 12 | 145 | 115 | 92 | 90 | 97 | 145 |
| 14 | 165 | 150 | 75 | 85 | 95 | 110 |
| 15 | 103 | 90 | 65 | 65 | 73 | 90 |
| 18 | 120 | 75 | 00 | 100 | 100 | - |
| 20 | 160 | 140 | 00 | 105 | 105 | 120 |
| 21 | 140 | 90 | 95 | 100 | 110 | 110 |
| 22 | 125 | 120 | 110 | 105 | 85 | 85 |
| 28 | 142 | 137 | 105 | 97 | 100 | 115 |
| 34 | 135 | 90 | 60 | 75 | 90 | 105 |
| 37 | 155 | 150 | 100 | 100 | 100 | 135 |
| 41 | 100 | 90 | 80 | 85 | 1 0 | - |

Antihypertensive Wirkung an spontan hypertensiven, wachen Ratten ("SH"-Ratten)

Der systolische Blutdruck wurde indirekt mit der "tail-cuff"-Methode an "SH"-Ratten (Wistar-Okamoto) [Arzneimittel-Forschung 6, 222 (1956)] bestimmt. Der Blutdruck der Ratten wurde vor der Behandlung und nach der Behandlung 24 Stunden lang stündlich gemessen. Die Prüfsubstanz wurde durch eine Sonde peroral verabreicht. Die Ergebnisse sind in Tabelle 3 zusammengefasst.

Tabelle 3

Antihypertensive Wirkung der Verbindungen der allgemeinen Formel I an wachen "SH"-Ratten

| Beispiel Nr. | Dosis (mg/kg) p.o. | Systolischer Blutdruck in mm Hg und Blutdrucksenkung in % | | | | | | | |
|---|---|---|---|---|---|---|---|---|---|
| | | $0^h$ | % | $2^h$ | % | $5^h$ | % | $24^h$ | % |
| 7 | 5 | 206 | 100 | 175,2 | -15 | 157,6 | -24 | 217,6 | + 5 |
| | 10 | 226 | 100 | 164,0 | -28 | 164,0 | -28 | 230,0 | + 1 |
| 9 | 10 | 190 | 100 | 166,6 | -13 | 166,6 | -13 | 192,0 | + 1 |
| 11 | 10 | 187,6 | 100 | 140 | -26 | 157,8 | -16 | 178,8 | - 5 |
| 12 | 10 | 190 | 100 | 164 | -14 | 166,0 | -13 | 190,8 | 0 |
| 14 | 10 | 198 | 100 | 165 | -17 | 164,5 | -17 | 206,8 | + 4 |
| 15 | 2.5 | 203,8 | 100 | 141,4 | -31 | 139,2 | -32 | 193,8 | - 5 |
| 18 | 10 | 200 | 100 | 179,2 | -11 | 178 | -11 | 192,6 | - 4 |
| 21 | 10 | 193,6 | 100 | 175,2 | -10 | 167,8 | -14 | 201,4 | + 4 |
| 22 | 2.5 | 193,9 | 100 | 142,2 | -27 | 144,0 | -26 | 191,4 | - 2 |
| 28 | 10 | 194 | 100 | 174,8 | -10 | 163,5 | -16 | 202,4 | + 4 |
| 34 | 10 | 224 | 100 | 170,0 | -25 | 182,0 | -19 | 216,0 | - 4 |
| 36 | 10 | 212 | 100 | 172,8 | -19 | 172,4 | -19 | 222,0 | + 4 |
| Nifedipin | 5 | 219,7 | 100 | 167,3 | -24 | 181,0 | -18 | 210,0 | - 8 |
| | 10 | 218,2 | 100 | 139,2 | -37 | 147,1 | -33 | 198,5 | -10 |

Antihypertensive Wirkung an Ratten mit renaler Hypertension ("RH"-Ratten)

Der Test wurde gemäss der Methode von Grollman [Proc. Soc. Exptl. Biol. Med., 57, 102 (1944)] an männlichen CFY-Ratten durchgeführt, die ein Körpergewicht von 120-150 g hatten. 5-6 Wochen nach der Operation erreichte der Blutdruck der Ratten 180-200 mm Hg.Die Prüfsubstanzen wurden peroral verabreicht. Die Ergebnisse sind in Tabelle 4 zusammengefasst.

Antihypertensive Wirkung an Ratten mit DOCA-hervorgerufener Hypertension ("DH"-Ratten)

Der Test wurde gemäss Cunning und Mitarbeitern [J. Pharm. Exp. Ther., 161, 88 (1981)] durchgeführt. Die Ergebnisse sind in Tabelle 5 zusammengefasst.

Die in Tabellen 2-5 zusammengefassten Ergebnisse zeigen, dass die erfindungsgemässen Verbindungen der allgemeinen Formel I signifikante antihypertensive Wirkung sowohl bei intraduodenaler als auch bei peroraler Verabreichung aufweisen; einige Verbindungen haben dieselbe Wirksamkeit wie die Vergleichssubstanz Nifedipin, andere sind sogar wirksamer.

Tabelle 4

Antihypertensive Wirkung der Verbindungen von Beispiel 7 und 34 an wachen "RH"-Ratten

| Beispiel Nr. | Dosis (mg/kg) p.o. | Systolischer Blutdruck in mm Hg und Blutdrucksenkung in % | | | | | | | |
|---|---|---|---|---|---|---|---|---|---|
| | | $0^h$ | % | $2^h$ | % | $5^h$ | % | $24^h$ | % |
| 7 | 5 | 206.6 | 100 | 177.7 | -15 | 188.6 | -13 | 213.3 | + 3 |
| | 10 | 198.8 | 100 | 138.8 | -31 | 150.0 | -25 | 212.2 | + 6 |
| 34 | 10 | 198.0 | 100 | 141.0 | -28 | 167.0 | -14 | 199.0 | 0 |
| Nifedipin | 5 | 173.5 | 100 | 141.0 | -19 | 147.5 | -15 | 173.5 | 0 |
| | 10 | 195.7 | 100 | 163.3 | -31 | 131.1 | -34 | 194.6 | - 1 |

Tabelle 5

Antihypertensive Wirkung der Verbindungen von Beispiel 7 und 34 an wachen, DOCA-hypertonischen Ratten

| Beispiel Nr. | Dosis (mg/kg) p.o. | Systolischer Blutdruck in mm Hg und Blutdrucksenkung in % | | | | | | | |
|---|---|---|---|---|---|---|---|---|---|
| | | $0^h$ | % | $2^h$ | % | $5^h$ | % | 24 h | % |
| 7 | 5 | 176.8 | 100 | 135.7 | -24 | 149.4 | -16 | 180.8 | + 2 |
| | 10 | 176.2 | 100 | 126.7 | -28 | 147.5 | -17 | 177.5 | 0 |
| 34 | 5 | 192.0 | 100 | 150.0 | -22 | 163.0 | -16 | 193.0 | 0 |
| | 10 | 189.0 | 100 | 146.0 | -23 | 134.0 | -30 | 186.0 | - 2 |

Die Calciumantagonistwirkung der erfindungsgemässen Verbindungen der allgemeinen Formel I wurde mit den folgenden Methoden in vitro bestimmt.

Calciumantagonistwirkung an einer isolierten Venenpräparation

Der Test wurde gemäss der Methode von M. Fiol de Cuneo und Mitarbeiter [Arch. Int. Pharmacodyn. Ther., 263, 28 (1983)] durchgeführt. Portale Venen wurden aus enthaupteten männlichen Ratten, die ein Körpergewicht von 300-350 g hatten, präpariert. Das länglich abgeschnittene Venensegment wurde in einer mit isotonischer Salzlösung gefüllten Organkammer (10 ml), unter einer ständigen Tension von 1 g aufgehängt. Sein oberes Ende wurde mit einem HSI-Kraftüberträger verbunden, und die isometrischen Kontraktionen wurden mit einem Beckman Graph registriert.

Nach einer Gleichgewichtsperiode in einer calcium-freien Krebs-Ringer-Bicarbonatlösung (KRB) am Anfang wurde die Vene 10 Minuten lang in eine Depolarisierungslösung (Calcium-freie KRB-Lösung, die 90 mM KCl enthält) gelegt. Um das kumulative Kontraktionsansprechen zu bestimmen, wurde dann $CaCl_2$ in Endkonzentrationen von 2,5 und 5,0 mM zugefügt. Jedes Medium wurde mittels Carbogengas mit Sauerstoff versehen, Temperatur und pH wurden, auf 37°C bzw. 7,4 eingestellt.

Um die Wirkung der geprüften Verbindungen auf die Kontraktion zu bestimmen, wurden die Venen 10 Minuten lang vor und nach der Zugabe von $Ca^{+2}$ zu der Depolarisierungslösung inkubiert. Jede Venenpräparation diente zu der Prüfung einer einzigen Konzentration der Prüfsubstanz.

Während des Versuchs wurde ein 100%-iges Ansprechen als die maximale Kontraktion der Kontroll-Calciumkurven angesehen. Die Kontraktionen nach Zugabe der Prüfsubstanz wurden als Prozent dieser Werte ausgedrückt. Die Konzentration, die eine 50%-ige Hemmung der maximalen Kontraktion im Vergleich zu der Kontrollkurve verursacht ($IC_{50}$, M), wurde aus den Konzentrations/Ansprech-Kurven bestimmt, indem die prozentuale Hemmung der Kontraktion gegen die Konzentration der Prüfsubstanz aufgetragen wurde. Während des ganzen Versuchs diente Nifedipin als Vergleichssubstanz. Die Ergebnisse sind in Tabelle 6 zusammengefasst.

Tabelle 6

Calciumantagonist-Wirkung der Verbindungen der allgemeinen Formel I

| Verbindung Beispiel Nr. | Hemmung des Calciumkanals $IC_{50}$, M |
|---|---|
| 7 | $5 \times 10^{-10}$ |
| 15 | $3 \times 10^{-9}$ |
| 34 | $2,5 \times 10^{-9}$ |
| Nifedipin (Vergleich) | $2 \times 10^{-9}$ |

Rezeptorbindung

Zwischen den strukturell unterschiedlichen Gruppen von Calcium-Antagonisten gibt es einige Pharmaka, die durch Wechselwirkung mit höchst spezifischen Rezeptorstellen der Sarkolemmamembran die Funktion der Calciumkanäle hemmen können. In der Serie der 1,4-Dihydropyridine kann der Ligandcharakter einer Verbindung durch die Analyse ihrer Wirkung auf die spezifische Bindung von $^3$H-Nitrendipin - ein wirksames Nifedipin-Analog -an das mikrosomale Herzpräparat geprüft werden [G. T. Bolger und Mitarbeiter: Biochem. Biophys. Res. Comm., 104, 1604 (1982)]. Die Kompetition der erfindungsgemässen Verbindungen für die 1,4-Dihydropyridin-Bindungsstellen wurde mit dieser Technik untersucht, und die Aktivität wurde mit der Konzentration ausgedrückt ($IC_{50}$), die dazu nötig war, um 50% des rezeptorgebundenen $^3$H-Nitrendipins unter den angewandten Testbedingungen abzudrängen. Die erhaltenen Ergebnisse sind in Tabelle 7 zusammengefasst.

Tabelle 7

Rezeptorbindung der Verbindungen der allgemeinen Formel I

| Verbindung Beispiel Nr. | $IC_{50}$ ($10^{-9}$ M) |
|---|---|
| 7 | 6,3 ± 0,05 |
| 15 | 15,5 ± 3,5 |
| 34 | 16,0 ± 1,6 |
| Nitrendipin | 1,25 ± 0,15 |
| Nifedipin (Vergleich) | 4,3 ± 0,04 |

Der niedrige submikromolare Bereich der $IC_{50}$-Werte weist auf die hohe Affinität der erfindungsgemässen Verbindungen zu den 1,4-Dihydropyridin-Bindungsstellen hin. Es sei auch erwähnt, dass die Aktivität der neuen Verbindungen ein wenig niedriger ist, als die der Vergleichssubstanz. Dies weist darauf hin, dass der Kopplungsmechanismus zwischen Rezeptorstimulation und biologischer Antwort im Falle der neuen Verbindungen aktiver sein kann.

Gemäss den Ergebnissen der pharmakologischen Prüfungen können die erfindungsgemässen Verbindungen der allgemeinen Formel I günstig für die Behandlung von pathologisch schweren Hypertensionsfällen eingesetzt werden. Es ist besonders günstig, dass sie die Herzfrequenz nicht erhöhen - vereinzelt tritt selbst Bradycardie auf - und keine ZNS-Wirkung besitzen, dagegen werden sie parenteral gut absorbiert, was hohe Bioverfügbarkeit sichert. Ihre Toxizität ist allgemein niedrig, daher besitzen sie einen hohen therapeutischen Index.

Für therapeutische Zwecke werden die erfindungsgemässen Wirkstoffe im allgemeinen in einer täglichen Dosis zwischen 0,05 mg/kg Körpergewicht und 2,0 mg/kg Körpergewicht, vorzugsweise zwischen 0,1 mg/kg und 0,5 mg/kg Körpergewicht, gegebenenfalls täglich auf mehrere Teile verteilt und mit Rücksicht auf die Resorptionsbedingungen, verwendet.

Zur therapeutischen Anwendung werden aus den erfindungsgemäßen Wirkstoffen zwechmäßig Arzneimittelpräparate in der Weise bereitet, daß sie mit in der Arzneimittelherstellung üblichen, zur enteralen oder parenteralen Applikation geeigneten nicht-toxischen inerten, festen und/oder flüssigen Träger-und/oder Hilfstoffen vermischt werden. Geeignete Trägerstoffe sind z. B. Wasser, Gelatine, Lactose, Stärke, Pektine, Magnesiumstearat, Stearinsäure, Talk und Pflanzenöle. Als Hilfstoffe können z. B. Konservierungsmittel und Netzmittel sowie Emulgier-, Dispergiermittel, Puffersubstanzen und Aromatisiermittel verwendet werden.

Die erfindungsgemässen 1,4-Di-(hydro)-pyridinderivate können mittels obiger Träger-und Hilfsstoffe in übliche Arzneimittelformen, zum Beispiel feste Arzneimittelformen, wie Tabletten, Kapseln, Pillen und Suppositorien, oder in flüssige Arzneimittelformen, wie wäßrige oder ölige Lösungen, Suspensionen, Emulsionen oder Sirupe oder in zu Injektionszwecken geeignete Lösungen, Suspensionen und Emulsionen überführt werden.

Die Erfindung wird an Hand der folgenden Beispiele näher erläutert.

Beispiel 1

4-(2-Acetamidophenyl)-2,6-dimethyl-3,5-bis(methoxycarbonyl)-1,4-dihydropyridin

In eine Lösung von 315 mg (1 mM) 4-(2-Aminophenyl)-2,6-dimethyl-3,5-bis(methoxycarbonyl)-1,4-dihydropyridin [J. Am. Chem. Soc., 71, 4003 (1949)] in 1 ml Dioxan und 1 ml Pyridin wird in einem Temperaturbereich von 0-10°C während 20 Minuten 0,18 g Acetylchlorid in 1 ml Dioxan eingetropft. Das Reaktionsgemisch wird 40 Minuten lang bei dieser Temperatur und 3 Stunden lang bei Raumtemperatur gerührt, dann wird es in 10 ml wässrige, 10gew.-%-ige Salzsäure geschüttet. Der gebildete Niederschlag wird abfiltriert, mit Wasser gewaschen und getrocknet. Ausbeute 350 mg(97,5%), Schmp. 284-285°C (Äthanol).

Das Verfahren von Beispiel 1 wird angewandt, um die folgenden, in Tabelle 8 zusammengefassten Verbindungen herzustellen.

Tabelle 8

Verbindungen der allgemeinen Formel I, worin $R_4$ einen Methylrest bedeutet

| Beispiel Nr. | $R_1$ | $R_2$ | $R_3$ | Stellung des Acylamids | Ausb. % | Schmp. °C |
|---|---|---|---|---|---|---|
| 2 | Acetyl | Methoxycarbonyl | Methyl | 3 | 86.5 | 232-3 |
| 3 | 4-Chlor-benzoyl | Methoxycarbonyl | Methyl | 3 | 76 | 250-2 |
| 4 | 4-Nitrobenzoyl | Methoxycarbonyl | Methyl | 3 | 57 | 272-4 |
| 5 | 4-Methylbenzoyl | Methoxycarbonyl | Methyl | 3 | 69 | 210-1 |
| 6 | 4-Chlor-benzoyl | Methoxycarbonyl | Methyl | 2 | 45 | 278-80 |
| 7 | 4-Nitrobenzoyl | Methoxycarbonyl | Methyl | 2 | 70 | 261-2 |
| 8 | 2-Nitrobenzoyl | Methoxycarbonyl | Methyl | 2 | 52 | 268-70 |
| 9 | Cinnamoyl | Methoxycarbonyl | Methyl | 2 | 41 | 144-6 |
| 10 | Fur-2-oyl | Methoxycarbonyl | Methyl | 2 | 32 | 283-5 |
| 11 | 4-Nitrocinnamoyl | Methoxycarbonyl | Methyl | 2 | 38 | 248-50 |
| 12 | Benzoyl | Methoxycarbonyl | Methyl | 2 | 38 | 257-9 |
| 13 | 3-Nitrobenzoyl | Methoxycarbonyl | Methyl | 2 | 40 | 281-3 |
| 14 | 4-Methoxybenzoyl | Methoxycarbonyl | Methyl | 2 | 32 | 235-6 |
| 15 | 4-Methylbenzoyl | Methoxycarbonyl | Methyl | 2 | 81 | 222-3 |
| 16 | 3,4-Dimethoxybenzoyl | Methoxycarbonyl | Methyl | 2 | 25 | 252-3 |
| 17 | 3,4,5-Trimethoxybenzoyl | Methoxycarbonyl | Methyl | 2 | 31 | 247-8 |
| 18 | Octanoyl | Methoxycarbonyl | Methyl | 2 | 45 | 99-100 |
| 19 | Cyclopropan-carbonyl | Methoxycarbonyl | Methyl | 2 | 67 | 224-6 |
| 20 | 3-Methylbenzoyl | Methoxycarbonyl | Methyl | 2 | 44 | 236-7 |
| 21 | 2-Methylbenzoyl | Methoxycarbonyl | Methyl | 2 | 59 | 231-2 |
| 22 | 4-Äthylbenzoyl | Methoxycarbonyl | Methyl | 2 | 31 | 222-3 |
| 23 | Phenylacetyl | Methoxycarbonyl | Methyl | 2 | 52 | 240-1 |
| 24 | Nicotinoyl | Methoxycarbonyl | Methyl | 2 | 75 | 238-40 |
| 25 | Isonicotinoyl | Methoxycarbonyl | Methyl | 2 | 45 | 241-2 |
| 26 | 3-Trifluor-methylbenzoyl | Methoxycarbonyl | Methyl | 2 | 33 | 215-6 |
| 27 | 4-Methylsulfonylbenzoyl | Methoxycarbonyl | Methyl | 2 | 32 | 228-30 |
| 28 | 4-Nitrobenzoyl | Methoxycarbonyl | 2-Methoxyethyl | 2 | 33 | 128-30 |
| 29 | 4-Nitrobenzoyl | Methoxycarbonyl | Cyclohexyl | 2 | 36 | 208-10 |
| 30 | 2-Methylthiobenzoyl | Methoxycarbonyl | Methyl | 2 | 80 | 255-6 |
| 31 | 2-Methylsulfinylbenzoyl | Methoxycarbonyl | Methyl | 2 | 17 | 306-8 |
| 32 | 4-Methylbenzoyl | t-Butoxycarbonyl | Methyl | 2 | 37 | 261-3 |

Beispiel 33

2,6-Dimethyl-3,5-bis(methoxycarbonyl)-4-(3-trifluoracetamidophenyl)-1,4-dihydropyridin

Ein Gemisch von 630 mg (2 mM) 4-(3-Aminophenyl)-2,6-dimethyl-3,5-bis(methoxycarbonyl)-1,4-dihydropyridin, 420 mg (4 mM) Trifluoressigsäureanhydrid und 0,18 g (23 mM) Pyridin wird bei 5 bis 10°C gerührt, dann werden 2,3 ml Pyridin tropfenweise, während 25 Minuten zugefügt. Das Rühren wird für weitere 24 Stunden bei Raumtemperatur fortgesetzt. Nachfolgend wird das Reaktionsgemisch wie in Beispiel 1 aufgearbeitet. Ausbeute 285 mg (35 %), Schmp. 231-2°C (Äthanol).

Mit dem Verfahren von Beispiel 33 werden folgende Verbindungen hergestellt:

Beispiel 34

2,6-Dimethyl-3,5-bis(methoxycarbonyl)-4-(2-trifluoracetamidophenyl)-1,4-dihydropyridin

Ausbeute 76 %, Schmp. 239-240°C.

Beispiel 35

2,6-Dimethyl-3,5-bis(äthoxycarbonyl)-4-(2-trifluoracetamidophenyl)-1,4-dihydropyridin

Ausbeute 37 %, Schmp. 214-5°C.

Beispiel 36

2,6-Dimethyl-3-methoxycarbonyl-5-(2-methoxyäthoxycarbonyl)-4-(2-trifluoracetamidophenyl)-1,4-dihydropyridin

Ausbeute 19 %, Schmp. 74-5°C.

Beispiel 37

2,6-Dimethyl-3-isopropoxycarbonyl-5-methoxycarbonyl-4-(2-trifluoracetamidophenyl)-1,4-dihydropyridin

Ausbeute 50 %, Schmp. 207-8°C.

Beispiel 38

2,6-Dimethyl-4-(3-äthoxycarbonylaminophenyl)-3,5-bis-(methoxycarbonyl)-1,4-dihydropyridin

Ein Gemisch von 500 mg 4-(3-Aminophenyl)-2,6-dimethyl-3,5-bis(methoxycarbonyl)-1,4-dihydropyridin, 5 ml Dichlormethan und 500 mg Diäthylpyrocarbonat wird 3 Stunden lang bei Raumtemperatur gerührt, über Nacht stehen gelassen und nachfolgend im Vakuum zur Trockne eingedampft.
Ausbeute 450 mg (73 %), Schmp. 183-5°C (Äthanol).

Mit dem Verfahren von Beispiel 38 wird folgende Verbindung hergestellt:

Beispiel 39

2,6-Dimethyl-4-(2-äthoxycarbonylamidophenyl)-3,5-bis-(methoxycarbonyl)-1,4-dihydropyridin

Ausbeute 32 %, Schmp. 182-3°C.

Beispiel 40

2,6-Dimethyl-4-(3-methansulfonylamidophenyl)-3,5-bis-(methoxycarbonyl)-1,4-dihydropyridin

Zu einer gerührten Lösung von 1,14 g (3,6 mM) 4-(3-Aminophenyl)-2,6-dimethyl-3,5-bis-(methoxycarbonyl)-1,4-dihydropyridin in 4 ml Pyridin werden innerhalb 30 Minuten bei 5°C tropfenweise 0,7 g (6,14 mM) Methansulfonylchlorid zugefügt, und das Rühren wird für weitere 24 Stunden bei Raumtemperatur fortgesetzt. Nachfolgend wird das Reaktionsgemisch in eine wäßrige, 4 gew.-%-ige Salzsäure-Lösung geschüttet, der erhaltene Niederschlag wird abfiltriert, mit Wasser gewaschen und dann in Äthanol unter Rückfluß zum Sieden erhitzt.
Ausbeute 1,2 g (86 %), Schmp. 225-6°C.

Mit dem Verfahren von Beispiel 40 werden folgende Verbindungen hergestellt:

Beispiel 41

2,6-Dimethyl-4-(2-methansulfonylamidophenyl)-3,5-bis-(methoxycarbonyl)-1,4-dihydropyridin

Ausbeute 43 %, Schmp. 224-5°C.

Beispiel 42

2,6-Dimethyl-3,5-bis(methoxycarbonyl)-4-[2-(4-nitrophenylsulfonamido)-phenyl]-1,4-dihydropyridin

Ausbeute 35 %, Schmp. 203-4°C.

Beispiel 43

2,6-Dimethyl-4-[2-(4-methylphenylsulfonamido)-phenyl]-3,5-bis(methoxycarbonyl)-1,4-dihydropyridin

Ausbeute 66 %, Schmp. 167-9°C.

Beispiel 44

2,6-Dimethyl-4-[2-(phenylsulfonamido)-phenyl]-3,5-bis(methoxycarbonyl)-1,4-dihydropyridin

Ausbeute 12 %, Schmp. 209-211°C.

Beispiel 45

2,6-Dimethyl-3-methoxycarbonyl-5-cyano-4-[2-(nitrobenzamido)-phenyl]-1,4-dihydropyridin

Zu einer Lösung von 0,85 g (3 mM) 5-Cyano-2,6-dimethyl-3-methoxycarbonyl-4-(2-aminophenyl)-1,4-dihydropyridin in 8,5 ml wasserfreiem Dioxan werden 0,4 ml (5 mM) Pyridin zugefügt. Die Lösung wird dann auf 15°C abgekühlt und mit einer Lösung von 0,6 g (3,3 mM) 4-Nitrobenzoylchlorid in 4 ml wasserfreiem Dioxan tropfenweise versetzt. Das Reaktionsgemisch wird 15 Stunden lang bei Raumtemperatur gerührt, dann in Wasser geschüttet. Die ausgefallenen Kristalle werden abfiltriert und aus einem Gemisch von Dimethylsulfoxid und Äthanol umkristallisiert.
Ausbeute 0,85 g (66 %), Schmp. 274-7°C.

5-Cyano-2,6-dimethyl-3-methoxycarbonyl-4-(2-aminophenyl)-1,4-dihydropyridin, das als Ausgangsmaterial verwendet worden ist, wird mit folgender Methode hergestellt:

Eine Lösung von 0,8 g (2,5 mM) 5-Cyano-2,6-dimethyl-3-methoxycarbonyl-4-(2-nitrophenyl)-1,4-dihydropyridin (DAS 2 658 804) in 80 ml Methanol wird bei atmosphärischem Druck, in Gegenwart von Pd-C-Katalysator der Hydrogenolyse unterworfen. Nach beendeter Reduktion wird der Katalysator abfiltriert und mit heissem Methanol gewaschen. Das Lösungsmittel wird abgedampft und der Rückstand aus Äthanol umkristallisiert.

Ausbeute 0,6 g (83 %), Schmp. 207-210°C.

Mit dem Verfahren von Beispiel 45 werden folgende, in Tabelle 9 zusammengefasste Verbindungen hergestellt.

## Tabelle 9

Verbindungen der allgemeinen Formel I, worin $R_4$ einen Methylrest bedeutet

| Beispiel Nr. | $R_1$ | $R_2$ | $R_3$ | Stellung des Acylamids | Ausb. % | Schmp. °C |
|---|---|---|---|---|---|---|
| 46 | 4-Chlor-benzoyl | Cyano | Methyl | 3 | 88 | 252-4 |
| 47 | 4-Nitrobenzoyl | Cyano | Methyl | 3 | 65 | 278-280 |
| 48 | 4-Nitrobenzoyl | Cyano | Äthyl | 3 | 67 | 271-3 |
| 49 | 4-Methoxybenzoyl | Cyano | Methyl | 2 | 37 | 129-131 |
| 50 | 4-Methoxybenzoyl | Cyano | Methyl | 3 | 40 | 226-8 |
| 51 | 4-Methylbenzoyl | Cyano | Methyl | 2 | 71 | 244-6 |
| 52 | 4-Methylbenzoyl | Cyano | Methyl | 3 | 70 | 233-5 |
| Verbindungen, die gemäss Beispiel Nr. 33 hergestellt wurden | | | | | | |
| 53 | Trifluor-acetyl | Cyano | Methyl | 3 | 75 | 229-231 |
| 54 | Trifluor-acetyl | Cyano | Äthyl | 3 | 76 | 226-8 |



BAD ORIGINAL

Beispiel 55

4-[2-(4-Aminobenzamido)-phenyl]-2,6-dimethyl-3,5-bis-(methoxycarbonyl)-1,4-dihydropyridin

2,8 g (60 mM) gemäß Beispiel 4 hergestelltes 4-[2-(4-Nitrobenzamido)-phenyl]-2,6-dimethyl-3,5-bis-(methoxycarbonyl)-1,4-dihydropyridin werden in 100 ml wasserfreiem Methanol gelöst und 2 Stunden lang in Gegenwart von 1,8 g 10 %-igem Pd-C Katalysator in 100 ml wasserfreiem Methanol hydriert. Nach beendeter Reaktion wird der Katalysator abfiltriert, dreimal mit 25 ml heissem Aceton, dann mit 25 ml Acetonitril gewaschen. Das Lösungsmittel wird im Vakuum abgedampft, der Rückstand dreimal mit Wasser gewaschen und nachfolgend getrocknet.
Ausbeute 2,4 g (91,5 %), Schmp. 266-7°C.

Beispiel 56

4-[2-(4-Acetamidobenzamido)-phenyl]-2,6-dimethyl-3,5-bis-(methoxycarbonyl)-1,4-dihydropyridin

Aus 4-(2-Aminophenyl)-2,6-dimethyl-3,5-bis-(methoxycarbonyl)-1,4-dihydropyridin und 4-Acetamidobenzoylchlorid ausgehend wird gemäß dem Verfahren von Beispiel 1 das Endprodukt hergestellt.
Ausbeute 79%, Schmp. 315-7°C.

Beispiel 57

4-[2-(4-Methansulfonylamidobenzamido)-phenyl]-2,6-dimethyl-3,5-bis-(methoxycarbonyl)-1,4-dihydropyridin

Aus 4-(2-Aminophenyl)-2,6-dimethyl-3,5-bis-(methoxycarbonyl)-1,4-dihydropyridin und 4-Methansulfonylamidobenzoylchlorid ausgehend wird gemäß dem Verfahren von Beispiel 1 das Endprodukt hergestellt.
Ausbeute 46%, Schmp. 309-11°C.

Herstellung von pharmazeutischen Präparaten

Beispiel 58

Herstellung von Tabletten

| Zusammensetzung (berechnet für 1000 Tabletten) | g |
|---|---|
| 2,6-Dimethyl-3,5-bis(methoxycarbonyl-4-/2-(trifluoracetamido)-phenyl/-1,4-dihydropyridin | 10 |
| Lactose | 185 |
| Mikrokristalline Cellulose | 25 |
| Talk | 5 |
| Maisstärke | 73 |
| Magnesiumstearat | 2 |
| Insgesamt: | 300 |

Die Komponenten werden vermischt, homogenisiert und zu Tabletten gepresst, die je 10 mg des Wirkstoffes enthalten.

Beispiel 59

Herstellung einer Injektionslösung

| Zusammensetzung (berechnet für 2 Liter Lösung) | | g |
|---|---|---|
| 2,6-Dimethyl-3,5-bis(methoxycarbonyl)-4-/2-(trifluoracetamido)-phenyl/-1,4-dihydropyridin | | 2 |
| Natriumchlorid | | 20 |
| Wasser für Injektionszwecke q.s. | ad | 2000 ml |

Die obigen Komponenten werden gelöst, in Ampullen gefüllt und sterilisiert. Eine Ampulle enthält 2 ml Lösung.



BAD ORIGINAL

**Ansprüche**

1.) 4-[N-(Substituierte)-aminophenyl]-substituierte 1,4-Di-(hydro)-pyridinderivate der allgemeinen Formel

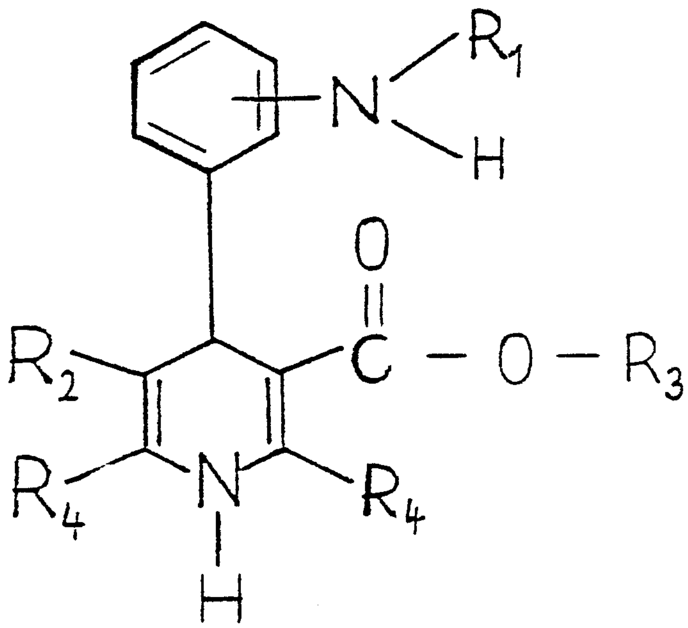

I ,

worin

$R_1$

für einen Alkanoylrest mit 2 bis 10 Kohlenstoffatomen, einen Cycloalkanoylrest mit 4 bis 7 Kohlenstoffatomen, einen Arylcarbonyl-beziehungsweise Aralkylencarbonylrest der allgemeinen Formel

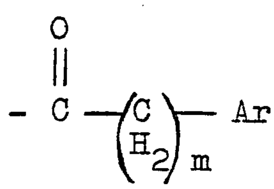

II ,

in welchletzterer

m

0 bis 3 ist,

die Alkylengruppe

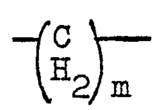

geradkettig oder verzweigt, gesättigt oder ungesättigt ist und

Ar

einen, gegebenenfalls durch ein Halogenatom, einen Alkylrest mit 1 bis 4 Kohlenstoffatom(en), 1 oder mehrere Alkoxyrest(e) mit 1 bis 4 Kohlenstoffatom(en), einen Alkylthiorest mit 1 bis 4 Kohlenstoffatom(en), einen Alkylsulfinylrest mit 1 bis 4 Kohlenstoffatom(en), einen Alkylsulfonylrest mit 1 bis 4 Kohlenstoffatom-(en), einen Trifluormethylrest, eine Nitrogruppe, eine Aminogruppe oder eine, gegebenenfalls durch einen Acetyl-, Trifluoracetyl-oder einen Methansulfonylrest substituierte, Aminogruppe substituierten, Arylrest oder einen ein Stickstoff-und/oder Sauerstoffatom aufweisenden aromatischen Rest darstellt,

einen Trifluoracetylrest, einen Alkoxycarbonylrest der allgemeinen Formel

$$-\overset{O}{\overset{\|}{C}}-O-R_5 \quad III,$$

in welchletzterer

$R_5$

einen Alkylrest mit 1 bis 4 Kohlenstoffatom(en) darstellt, oder

einen Alkylsulfonyl-oder Phenylsulfonylrest der allgemeinen Formel

$$-\underset{O_2}{S}-R_6 \quad IV,$$

in welchletzterer

$R_6$

einen Alkylrest mit 1 bis 4 Kohlenstoffatom(en), oder einen, gegebenenfalls durch einen Alkylrest mit 1 bis 4 Kohlenstoffatom(en) oder eine Nitrogruppe substituierten, Phenylrest darstellt,

steht,

$R_2$

eine Cyangruppe oder einen geradkettigen oder verzweigten Alkoxycarbonylrest mit 2 bis 4 Kohlenstoffatomen bedeutet,

$R_3$

für einen geradkettigen oder verzweigten Alkylrest mit 1 bis 4 Kohlenstoffatom(en), einen Cycloalkylrest mit 5 bis 7 Kohlenstoffatomen oder einen Alkoxyalkylrest mit 2 bis 6 Kohlenstoffatomen steht und

$R_4$

einen Alkylrest mit 1 bis 4 Kohlenstoffatomen bedeutet,

einschließlich ihrer racemischen und optisch aktiven Formen und Gemische sowie ihre Säureadditionssalze.

2.) 1,4-Di-(hydro)-pyridinderivate nach Anspruch 1, dadurch gekennzeichnet, daß der Alkanoylrest, für den $R_1$ stehen kann, ein solcher mit 2 bis 9 Kohlenstoffatomen ist, beziehungsweise der Cycloalkanoylrest, für den $R_1$ stehen kann, ein solcher mit 4 bis 6 Kohlenstoffatomen ist, beziehungsweise der Arylcarbonylbeziehungsweise Aralkylencarbonylrest der allgemeinen Formel II, für den $R_1$ stehen kann, ein solcher, bei welchem m 0 bis 2 ist und/oder der Arylrest, für den Ar stehen kann, ein Phenylrest ist und/oder ein solcher, bei welchem der Alkylrest, der beziehungsweise die Alkoxyrest(e), der Alkylthiorest, der Alkylsulfinylrest beziehungsweise der Alkylsulfonylrest, durch welche[n] er substituiert sein kann, ein solche[r] mit 1 oder 2 Kohlenstoffatom(en) ist be ziehungsweise sind, beziehungsweise der ein Stickstoff-und/oder Sauerstoffatom aufweisende aromatische Rest, für den Ar stehen kann, ein solcher mit 5 oder 6 Ringgliedern ist, beziehungsweise der Alkoxycarbonylrest der allgemeinen Formel III, für den $R_1$ stehen kann, ein solcher, dessen $R_5$ einen Alkylrest mit 1 oder 2 Kohlenstoffatom(en) darstellt, ist, beziehungsweise der Alkylsulfonylrest der allgemeinen Formel IV, für den $R_1$ stehen kann, ein solcher, bei welchem der Alkylrest, für den $R_6$ stehen kann, ein solcher mit 1 oder 2 Kohlenstoffatom(en) ist, beziehungsweise der Phenylsulfonylrest der allgemeinen Formel IV, für den $R_1$ stehen kann, ein solcher, bei welchem der Alkylrest, durch den der Phenylrest, für den $R_6$ stehen kann, substituiert sein kann, ein solcher mit 1 oder 2 Kohlenstoffatom(en) ist.

3.) 1,4-Di-(hydro)-pyridinderivate nach Anspruch 1 oder 2, dadurch gekennzeichnet, daß der Alkoxycarbonylrest, für den $R_2$ stehen kann, ein solcher mit 2 oder 3 Kohlenstoffatomen ist.

4.) 1,4-Di-(hydro)-pyridinderivate nach Anspruch 1 bis 3, dadurch gekennzeichnet, daß der beziehungsweise die Alkylrest(e), für welche[n] $R_3$ und/oder $R_4$ stehen kann beziehungsweise können, ein solche[r] mit 1 bis 3, insbesondere 1 oder 2 Kohlenstoffatom(en) ist beziehungsweise sind, beziehungsweise der Cycloalkylrest, für den $R_3$ stehen kann, ein solcher mit 5 oder 6 Kohlenstoffatomen ist, beziehungsweise der Alkoxyalkylrest, für den $R_3$ stehen kann, ein solcher mit 2 oder 3 Kohlenstoffatomen ist.

5.) 1,4-Di-(hydro)-pyridinderivate nach Anspruch 1 bis 4, dadurch gekennzeichnet, daß

$R_1$

für einen Alkanoylrest mit 2 bis 10 Kohlenstoffatomen oder einen Cycloalkanoylrest mit 4 bis 7 Kohlenstoffatomen steht und

$R_2$, $R_3$ und $R_4$

die in den Ansprüchen 1, 3 oder 4 angegebenen Bedeutungen haben.

6.) 1,4-Di-(hydro)-pyridinderivate nach Anspruch 1 bis 4, dadurch gekennzeichnet, daß

$R_1$

für einen Rest der allgemeinen Formel II,

in welchletzterer

Ar

einen, gegebenenfalls durch ein Halogenatom, einen Alkylrest mit 1 bis 4 Kohlenstoffatom(en), 1 oder mehrere Alkoxyrest(e) mit 1 bis 4 Kohlenstoffatom(en), einen Alkylthiorest mit 1 bis 4 Kohlenstoffatom(en), einen Alkylsulfinylrest mit 1 bis 4 Kohlenstoffatom(en), einen Alkylsulfonylrest mit 1 bis 4 Kohlenstoffatom-

(en), einen Trifluormethylrest, eine Nitrogruppe, eine Aminogruppe oder eine, gegebenenfalls durch einen Acetyl-, Trifluoracetyl-oder einen Methansulfonylrest substituierte, Aminogruppe substituierten, Phenylrest oder einen ein Stickstoff-und/oder Sauerstoffatom aufweisenden aromatischen Rest darstellt und
m, $R_2$, $R_3$ und $R_4$
die in den Ansprüchen 1 bis 4 angegebenen Bedeutungen haben.

7.) 1,4-Di-(hydro)-pyridinderivate nach Anspruch 1 bis 4, dadurch gekennzeichnet, daß
$R_1$
für einen Trifluoracetylrest steht und
$R_2$, $R_3$ und $R_4$
die in den Ansprüchen 1, 3 oder 4 angegebenen Bedeutungen haben.

8.) 2,6-Di-[methyl]-3,5-bis-[methoxycarbonyl]-4-[2'-(4"-<nitro> -benzamido)-phenyl]-1,4-di-[hydro]-pyridin,
2,6-Di-[methyl]-3,5-bis-[methoxycarbonyl]-4-[2'-(4"-<methyl> -benzamido)-phenyl]-1,4-di-[hydro]-pyridin,
2,6-Di-[methyl]-3,5-bis-[methoxycarbonyl]-4-[2'-(4"-<äthyl> -benzamido)-phenyl]-1,4-di-[hydro]-pyridin und
2,6-Di-[methyl]-3,5-bis-[methoxycarbonyl]-4-[2'-(trifluoracetamido)-phenyl]-1,4-di-[hydro]-pyridin
einschließlich ihrer racemischen und optisch aktiven Formen und Gemische sowie ihre Säureadditionssalze.

9.) Verfahren zur Herstellung der 1,4-Di-(hydro)-pyridinderivate nach Anspruch 1 bis 8, dadurch gekennzeichnet, daß man 4-(aminophenyl)-substituierte 1,4-Di-(hydro)-pyridinderivate der allgemeinen Formel

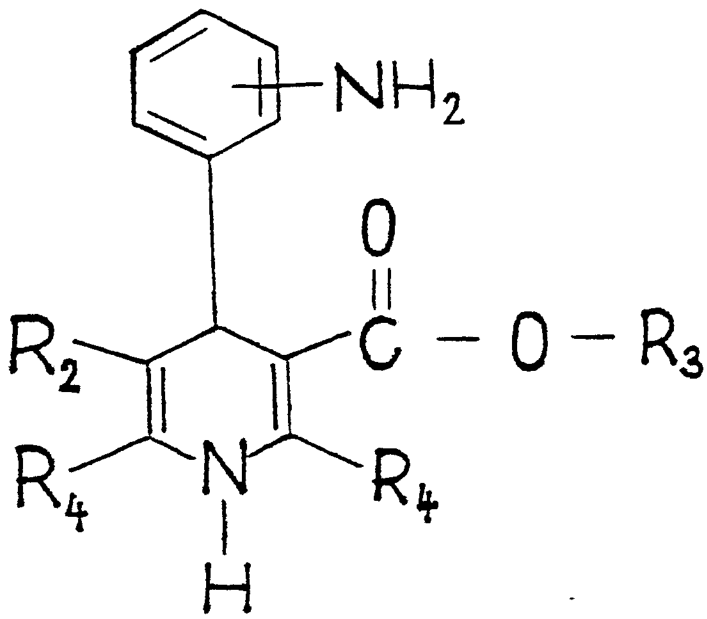

V ,

worin $R_2$ , $R_3$ und $R_4$ die in den Ansprüchen 1, 3 oder 4 angegebenen Bedeutungen haben, mit Verbindungen der allgemeinen Formel
$R_1$ -X VI ,
worin
$R_1$
die in den Ansprüchen 1, 2 oder 5 bis 7 angegebenen Bedeutungen hat und
X
eine zum Einführen einer Gruppe $R_1$ in das Molekül geeignete abspaltbare Gruppe darstellt,
umsetzt und gegebenenfalls die erhaltenen 1,4-Di-(hydro)-pyridinderivate der allgemeinen Formel I, bei welchen $R_1$ für einen Arylcarbonylrest der allgemeinen Formel II mit m = 0, bei welchem Ar einen durch eine Nitrogruppe substituierten Arylrest bedeutet, steht, in an sich bekannter Weise reduziert, worauf man in an sich bekannter Weise gegebenenfalls die erhaltenen 1,4-Di-(hydro)-pyridinderivate der allgemeinen Formel I in ihre Säureadditionssalze überführt und/oder gegebenenfalls die erhaltenen Säureadditionssalze der 1,4-Di-(hydro)-pyridinderivate der allgemeinen Formel I in die entsprechenden freien Basen und/oder andere Säureadditionssalze überführt und/oder gegebenenfalls die erhaltenen 1,4-Di-(hydro)-pyridinderivate der allgemeinen Formel I beziehungsweise ihre Säureadditionssalze in ihre optisch aktiven Formen spaltet und/oder gegebenenfalls die optisch aktiven Antipoden der 1,4-Di-(hydro)-pyridinderivate der allgemeinen Formel I beziehungsweise ihrer Säureadditionssalze racemisiert.

10.) Arzneimittel, gekennzeichnet durch einen Gehalt an 1 oder mehr 1,4-Di-(hydro)-pyridinderivaten nach Anspruch 1 bis 8 als Wirkstoff(en), zweckmäßig zusammen mit 1 oder mehr pharmazeutisch üblichen Träger-und/oder Hilfsstoff(en).

Patentansprüche für die folgenden Vertragsstaaten: AT, ES.

1.) Verfahren zur Herstellung von 4-[N-(substituierten)-aminophenyl]-substituierten 1,4-Di-(hydro)-pyridinderivaten der allgemeinen Formel

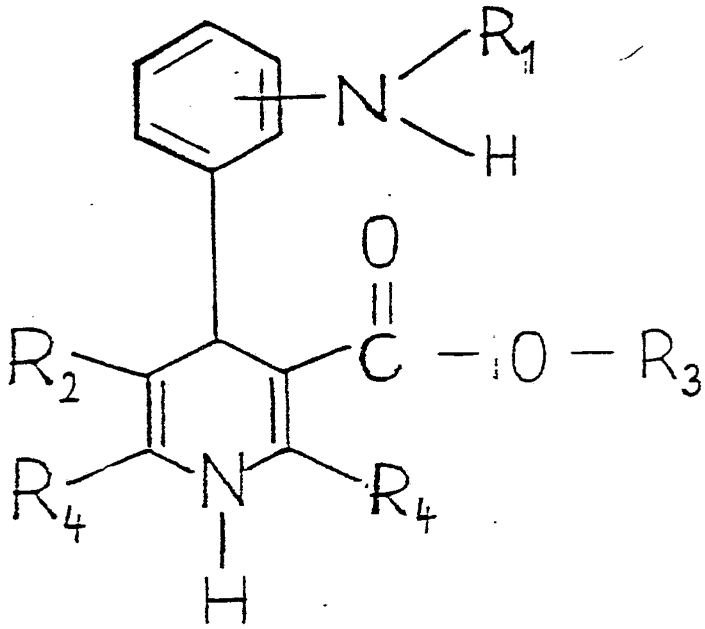

I ,

worin

$R_1$

für einen Alkanoylrest mit 2 bis 10 Kohlenstoffatomen, einen Cycloalkanoylrest mit 4 bis 7 Kohlenstoffatomen, einen Arylcarbonyl-beziehungsweise Aralkylencarbonylrest der allgemeinen Formel

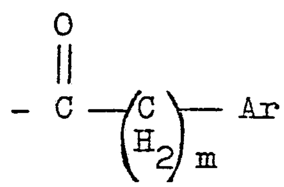

II ,

in welchletzterer

m

0 bis 3 ist,

die Alkylengruppe

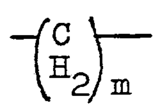

geradkettig oder verzweigt, gesättigt oder ungesättigt ist und

Ar

einen, gegebenenfalls durch ein Halogenatom, einen Alkylrest mit 1 bis 4 Kohlenstoffatom(en), 1 oder mehrere Alkoxyrest(e) mit 1 bis 4 Kohlenstoffatom(en), einen Alkylthiorest mit 1 bis 4 Kohlenstoffatom(en), einen Alkylsulfinylrest mit 1 bis 4 Kohlenstoffatom(en), einen Alkylsulfonylrest mit 1 bis 4 Kohlenstoffatom(en), einen Trifluormethylrest, eine Nitrogruppe, eine Aminogruppe oder eine, gegebenenfalls durch einen

Acetyl-, Trifluoracetyl-oder einen Methansulfonylrest substituierte, Aminogruppe substituierten, Arylrest oder einen ein Stickstoff-und/oder Sauerstoffatom aufweisenden aromatischen Rest darstellt,
einen Trifluoracetylrest, einen Alkoxycarbonylrest der allgemeinen Formel

$$-\overset{\overset{O}{\|}}{C}-O-R_5 \quad III,$$

in welchletzterer
$R_5$
einen Alkylrest mit 1 bis 4 Kohlenstoffatom(en) darstellt, oder
einen Alkylsulfonyl-oder Phenylsulfonylrest der allgemeinen Formel

$$-\underset{O_2}{S}-R_6 \quad IV,$$

in welchletzterer
$R_6$
einen Alkylrest mit 1 bis 4 Kohlenstoffatom(en), oder einen, gegebenenfalls durch einen Alkylrest mit 1 bis 4 Kohlenstoffatom(en) oder eine Nitrogruppe substituierten, Phenylrest darstellt,
steht,
$R_2$
eine Cyangruppe oder einen geradkettigen oder verzweigten Alkoxycarbonylrest mit 2 bis 4 Kohlenstoffatomen bedeutet,
$R_3$
für einen geradkettigen oder verzweigten Alkylrest mit 1 bis 4 Kohlenstoffatom(en), einen Cycloalkylrest mit 5 bis 7 Kohlenstoffatomen oder einen Alkoxyalkylrest mit 2 bis 6 Kohlenstoffatomen steht und
$R_4$
einen Alkylrest mit 1 bis 4 Kohlenstoffatomen bedeutet,
einschließlich ihrer racemischen und optisch aktiven Formen und Gemische sowie ihrer Säureadditionssalze, dadurch gekennzeichnet, daß man 4-(aminophenyl)-substituierte 1,4-Di-(hydro)-pyridinderivate der allgemeinen Formel

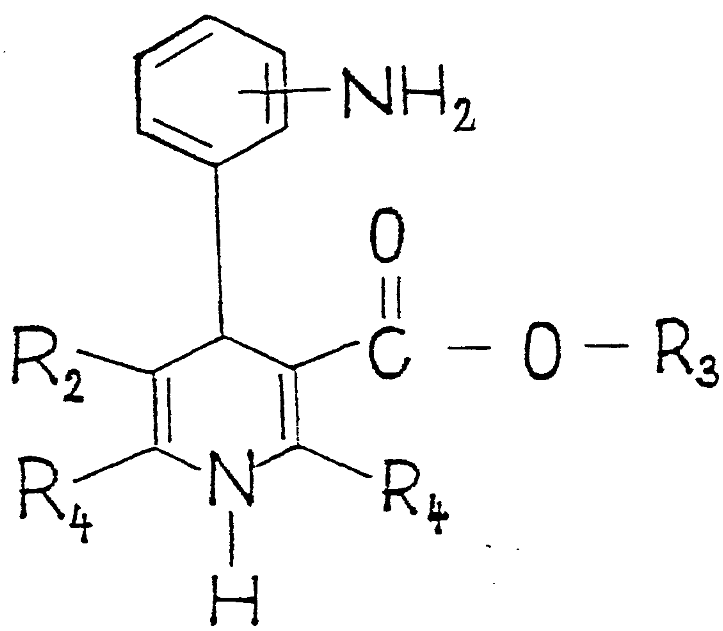

V ,

worin $R_2$ , $R_3$ und $R_4$ die oben angegebenen Bedeutungen haben, mit Verbindungen der allgemeinen Formel
$R_1$ -X VI ,
worin
$R_1$
die oben angegebenen Bedeutungen hat und
X
eine zum Einführen einer Gruppe $R_1$ in das Molekül geeignete abspaltbare Gruppe darstellt,

umsetzt und gegebenenfalls die erhaltenen 1,4-Di-(hydro)-pyridinderivate der allgemeinen Formel I, bei welchen $R_1$ für einen Arylcarbonylrest der allgemeinen Formel II mit m = 0, bei welchem Ar einen durch eine Nitrogruppe substituierten Arylrest bedeutet, steht, in an sich bekannter Weise reduziert, worauf man in an sich bekannter Weise gegebenenfalls die erhaltenen 1,4-Di-(hydro)-pyridinderivate der allgemeinen Formel I in ihre Säureadditionssalze überführt und/oder gegebenenfalls die erhaltenen Säureadditionssalze der 1,4-Di-(hydro)-pyridinderivate der allgemeinen Formel I in die entsprechenden freien Basen und/oder andere Säureadditionssalze überführt und/oder gegebenenfalls die erhaltenen 1,4-Di-(hydro)-pyridinderivate der allgemeinen Formel I beziehungsweise ihre Säureadditionssalze in ihre optisch aktiven Formen spaltet und/oder gegebenenfalls die optisch aktiven Antipoden der 1,4-Di-(hydro)-pyridinderivate der allgemeinen Formel I beziehungsweise ihrer Säureadditionssalze racemisiert.

2.) Verfahren zur Herstellung der 1,4-Di-(hydro)-pyridinderivate nach Anspruch 1, dadurch gekennzeichnet, daß man solche, bei welchen der Alkanoylrest, für den $R_1$ stehen kann, ein solcher mit 2 bis 9 Kohlenstoffatomen ist, beziehungsweise der Cycloalkanoylrest, für den $R_1$ stehen kann, ein solcher mit 4 bis 6 Kohlenstoffatomen ist, beziehungsweise der Arylcarbonylbeziehungsweise Aralkylencarbonylrest der allgemeinen Formel II, für den $R_1$ stehen kann, ein solcher, bei welchem m 0 bis 2 ist und/oder der Arylrest, für den Ar stehen kann, ein Phenylrest ist und/oder ein solcher, bei welchem der Alkylrest, der beziehungsweise die Alkoxyrest(e), der Alkylthiorest, der Alkylsulfinylrest beziehungsweise der Alkylsulfonylrest, durch welche[n] er substituiert sein kann, ein solche[r] mit 1 oder 2 Kohlenstoffatom(en) ist beziehungsweise sind, beziehungsweise der ein Stickstoff-und/oder Sauerstoffatom aufweisende aromatische Rest, für den Ar stehen kann, ein solcher mit 5 oder 6 Ringgliedern ist, beziehungsweise der Alkoxycarbonylrest der allgemeinen Formel III, für den $R_1$ stehen kann, ein solcher, dessen $R_5$ einen Alkylrest mit 1 oder 2 Kohlenstoffatom(en) darstellt, ist, beziehungsweise der Alkylsulfonylrest der allgemeinen Formel IV, für den $R_1$ stehen kann, ein solcher, bei welchem der Alkylrest, für den $R_6$ stehen kann, ein solcher mit 1 oder 2 Kohlenstoffatom(en) ist, beziehungsweise der Phenylsulfonylrest der allgemeinen Formel IV, für den $R_1$ stehen kann, ein solcher, bei welchem der Alkylrest, durch den der Phenylrest, für den $R_6$ stehen kann, substituiert sein kann, ein solcher mit 1 oder 2 Kohlenstoffatom(en) ist,herstellt.

3.) Verfahren zur Herstellung der 1,4-Di-(hydro)-pyridinderivate nach Anspruch 1 oder 2, dadurch gekennzeichnet, daß man solche, bei welchen der Alkoxycarbonylrest, für den $R_2$ stehen kann, ein solcher mit 2 oder 3 Kohlenstoffatomen ist , herstellt.

4.) Verfahren zur Herstellung der 1,4-Di-(hydro)-pyridinderivate nach Anspruch 1 bis 3, dadurch gekennzeichnet, daß man solche, bei welchen der beziehungsweise die Alkylrest(e), für welche[n] $R_3$ und/oder $R_4$ stehen kann beziehungsweise können, ein solche[r] mit 1 bis 3, insbesondere 1 oder 2 Kohlenstoffatom(en) ist beziehungsweise sind, beziehungsweise der Cycloalkylrest, für den $R_3$ stehen kann, ein solcher mit 5 oder 6 Kohlenstoffatomen ist, beziehungsweise der Alkoxyalkylrest, für den $R_3$ stehen kann, ein solcher mit 2 oder 3 Kohlenstoffatomen ist, herstellt.

5.) Verfahren zur Herstellung der 1,4-Di-(hydro)-pyridinderivate nach Anspruch 1 bis 4, dadurch gekennzeichnet, daß man solche, bei welchen

$R_1$

für einen Alkanoylrest mit 2 bis 10 Kohlenstoffatomen oder einen Cycloalkanoylrest mit 4 bis 7 Kohlenstoffatomen steht und

$R_2$ , $R_3$ und $R_4$

die oben angegebenen Bedeutungen haben, herstellt.

6.) Verfahren zur Herstellung der 1,4-Di-(hydro)-pyridinderivate nach Anspruch 1 bis 4, dadurch gekennzeichnet, daß man solche, bei welchen

$R_1$

für einen Rest der allgemeinen Formel II,

in welchletzterer

Ar

einen, gegebenenfalls durch ein Halogenatom, einen Alkylrest mit 1 bis 4 Kohlenstoffatom(en), 1 oder mehrere Alkoxyrest(e) mit 1 bis 4 Kohlenstoffatom(en), einen Alkylthiorest mit 1 bis 4 Kohlenstoffatom(en), einen Alkylsulfinylrest mit 1 bis 4 Kohlenstoffatom(en), einen Alkylsulfonylrest mit 1 bis 4 Kohlenstoffatom(en), einen Trifluormethylrest, eine Nitrogruppe, eine Aminogruppe oder eine, gegebenenfalls durch einen Acetyl-, Trifluoracetyl-oder einen Methansulfonylrest substituierte, Aminogruppe substituierten, Phenylrest oder einen ein Stickstoff-und/oder Sauerstoffatom aufweisenden aromatischen Rest darstellt und

m, $R_2$, $R_3$ und $R_4$

die oben angegebenen Bedeutungen haben, herstellt.

7.) Verfahren zur Herstellung der 1,4-Di-(hydro)-pyridinderivate nach Anspruch 1 bis 4, dadurch gekennzeichnet, daß man solche, bei welchen
$R_1$
für einen Trifluoracetylrest steht und
$R_2$ , $R_3$ und $R_4$
die oben angegebenen Bedeutungen haben, herstellt.

8.) Verfahren nach Anspruch 1 bis 7 zur Herstellung von
2,6-Di-[methyl]-3,5-bis-[methoxycarbonyl]-4-[2'-(4''-<nitro> -benzamido)-phenyl]-1,4-di-[hydro]-pyridin,
2,6-Di-[methyl]-3,5-bis-[methoxycarbonyl]-4-[2'-(4''-<methyl> -benzamido)-phenyl]-1,4-di-[hydro]-pyridin,
2,6-Di-[methyl]-3,5-bis-[methoxycarbonyl]-4-[2'-(4''-<äthyl> -benzamido)-phenyl]-1,4-di-[hydro]-pyridin und
2,6-Di-[methyl]-3,5-bis-[methoxycarbonyl]-4-[2'-(trifluoracetamido)-phenyl]-1,4-di-[hydro]-pyridin
einschließlich ihrer racemischen und optisch aktiven Formen und Gemische sowie ihrer Säureadditionssalze.

Europäisches Patentamt

# EUROPÄISCHER RECHERCHENBERICHT

Nummer der Anmeldung
EP 87113539.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE - A - 1 670 827 (BAYER)<br>* Ansprüche 1,2; Seite 2, Zeilen 12-24 *<br>-- | 1,10 | C 07 D 211/90<br>C 07 D 401/12<br>C 07 D 405/12<br>A 61 K 31/44<br>A 61 K 31/455 |
| Y | DE - A - 2 218 644 (BAYER)<br>* Ansprüche 1,3; Seite 25, Zeile 1 - Seite 26, Zeile 6; Beispiel 21 *<br>---- | 1,10 | |
| | | | **RECHERCHIERTE SACHGEBIETE (Int. Cl.4)** |
| | | | C 07 D 211/00<br>C 07 D 401/00<br>C 07 D 405/00 |

Der vorliegende Recherchenbericht wurde für alle Patentansprüche erstellt.

| Recherchenort | Abschlußdatum der Recherche | Prüfer |
|---|---|---|
| WIEN | 18-12-1987 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument